# DEMANDE DE BREVET EUROPEEN

(11) **EP 3 957 298 A1**
(43) Date de publication de la demande: **23.02.2022**
(21) Numéro de dépôt: 21191968.3
(22) Date de dépôt: 18.08.2021
(51) Int. Cl.: A61K 8/92, A61Q 5/12, A61Q 19/00

(54) **PROCEDE DE PREPARATION DE BEURRES**

(30) Priorité: 19.08.2020 FR 2008567
(71) Demandeur: Naturochim, 91190 Gif-sur-Yvette (FR)
(72) Inventeur: COUMANNE, Claudine, 91190 Gif-sur-Yvette (FR)
(74) Mandataire: Vial, Lionel

(57) **Abrégé**

La présente invention concerne un beurre comprenant une huile végétale et ladite huile végétale hydrogénée.

## Description

### Domaine de l'invention

La présente invention concerne des beurres destinés à être utilisés dans des compositions cosmétiques, le procédé de préparation de ces beurres ainsi que les compositions cosmétiques comprenant ces beurres.

### Arrière-plan technique

Aujourd'hui, la demande des consommateurs en matière de produits cosmétiques s'oriente vers davantage de naturel et de produits d'origine biologique. Les consommateurs sont ainsi plus regardants sur les composants de leurs produits d'hygiène et cosmétiques ce qui entraine une croissance des ventes des produits cosmétiques biologiques de plus de 5% chaque année.

Parmi les ingrédients qui connaissent un vrai succès on retrouve les huiles végétales. Les huiles végétales sont extraites de graines ou de fruits et présentent des propriétés nourrissantes et protectrices pour la peau et les cheveux.

De plus, les huiles végétales peuvent être transformées en beurres qui présentent des propriétés similaires aux huiles végétales. La différence par rapport aux huiles réside dans le fait qu'ils sont solides à température ambiante et redeviennent liquides lorsqu'on les chauffe, ils ont donc l'avantage d'apporter à la fois les bienfaits des huiles et une texture permettant d'obtenir des produits finis à galéniques différentes.

De manière générale, les beurres sont préparés par hydrogénation d'une huile bon marché, telle que l'huile de tournesol et l'ajout d'une autre huile végétale à cette huile hydrogénée afin d'obtenir la texture d'un beurre. A titre d'exemple, on parlera de beurre d'abricot si l'huile végétale ajoutée à l'huile hydrogénée est l'huile d'abricot.

L'hydrogénation est le procédée par lequel de l'hydrogène est ajouté aux points d'insaturation d'un acide gras. L'huile est mise à réagir avec de l'hydrogène à température et pression élevées en présence d'un catalyseur. L'hydrogénation peut être complète ou non, aboutissant à une huile hydrogénée, de consistance solide, ou partiellement hydrogénée, de consistance semi-solide.

Toutefois, l'hydrogénation à haute température et pression entraine la dégradation des huiles végétales sensibles à la température et la formation de sous-produits dus au cracking ou à la condensation qui ne sont pas souhaités dans des produits cosmétiques. En outre, la méthode de préparation des beurres décrite ci-dessus a pour inconvénient que le profil des acides gras du beurre est moins typique de celui de l'huile végétale ajoutée.

Il existe donc un besoin de fournir un procédé d'obtention de beurre à partir d'une seule huile végétale afin d'obtenir un profil d'acide gras typique de l'huile végétale utilisée et étant plus respectueux de cette huile.

### Résumé de l'invention

La présente invention découle de la mise en évidence par les inventeurs qu'un beurre préparé à partir d'une seule huile végétale présente des propriétés intéressantes, en particulier un profil d'acides gras typique de ladite huile végétale, pour une utilisation dans des compositions cosmétiques.

Ainsi, la présente invention concerne un beurre comprenant une huile végétale et ladite huile végétale hydrogénée.

La présente invention concerne également un beurre constitué d'une huile végétale, de ladite huile végétale hydrogénée et de vitamine E.

La présente invention concerne également un procédé de préparation de beurre destiné à être utilisé dans des compositions cosmétiques, à partir d'une huile végétale, comprenant les étapes suivantes :
- une étape d'hydrogénation de l'huile végétale ;
- une étape d'ajout de l'huile végétale à l'huile végétale hydrogénée de sorte à obtenir un beurre ;
- éventuellement une étape de désodorisation.

La présente invention concerne également un beurre destiné à être utilisé dans des compositions cosmétiques susceptible d'être préparé ou préparé selon le procédé défini ci-dessus.

La présente invention concerne également une composition cosmétique comprenant au moins un beurre tel que défini ci-dessus ou préparé par le procédé tel que défini ci-dessus.

### Description détaillée de l'invention

### Beurre

De préférence, le beurre selon l'invention est un beurre d'origine végétale. L'expression « beurre d'origine végétale » selon l'invention désigne un beurre obtenu à partir d'une source, notamment une huile, végétale et qui a une consistance solide à température ambiante, soit à une température comprise entre 15 et 25°C à une pression de 1 atmosphère.

De préférence, le beurre selon l'invention est d'origine naturelle. On entend par beurre d'origine naturelle selon l'invention, un beurre constitué uniquement d'ingrédients d'origine naturelle.

De préférence, le beurre selon l'invention est biologique. On entend par beurre biologique selon l'invention, un beurre étant issu au moins à 95% de matière issue de l'agriculture biologique.

Comme on l'entend ici, le beurre selon l'invention n'est pas une émulsion. On entend par «émulsion» un mélange hétérogène de deux substances liquides non miscibles. Ainsi, le beurre selon l'invention ne comprend essentiellement pas d'eau ou de phase hydrophile.

De préférence, le beurre selon l'invention est constitué d'une huile végétale, de ladite huile végétale hydrogénée et éventuellement de vitamine E.

De préférence également, le beurre selon l'invention ne comprend pas d'ajout d'au moins un insaponifiable. On entend par «insaponifiable» d'une huile végétale la fraction résiduelle insoluble dans l'eau après saponification de l'huile végétale.

A titre d'exemple de beurre selon l'invention, il est possible de citer le beurre d'amande, le beurre d'abricot, le beurre d'argan, le beurre d'avocat, le beurre de noix de coco, le beurre d'olive, le beurre de macadamia, le beurre de jojoba, le beurre de noisette, le beurre d'argousier, le beurre de figue, le beurre de framboise, le beurre de sésame, le beurre d'avoine, le beurre de mirabelle, le beurre de prune, le beurre de pistache, le beurre de chanvre, et le beurre de date du désert.

De préférence, le beurre selon l'invention est sélectionné dans le groupe constitué du beurre d'amande, du beurre d'abricot, du beurre d'argan, du beurre d'avocat, du beurre de noix de coco, du beurre de prune, du beurre de pistache, du beurre d'avoine et du beurre d'olive.

De préférence, le beurre selon l'invention comprend une huile végétale et de l'huile végétale hydrogénée. Comme cela apparaîtra clairement à la personne du métier, selon l'invention, l'huile végétale et l'huile végétale hydrogénée sont identiques sauf en ce qui concerne l'hydrogénation ; en particulier l'huile végétale hydrogénée selon l'invention dérive de l'huile végétale par hydrogénation de cette dernière. Avantageusement, l'huile végétale et ladite huile végétale hydrogénée selon l'invention se mélangent plus intimement que deux huiles provenant de sources différentes, il en résulte un beurre plus stable qui a moins tendance à déphaser que les beurres obtenus à partir d'un mélange d'huiles. Avantageusement également, l'utilisation d'une seule huile végétale pour la fabrication de beurre permet d'obtenir un beurre ayant des caractéristiques proches de celles de l'huile végétale utilisée. En particulier, les beurres obtenus à partir d'une même huile présentent un profil d'acides gras typique de l'huile utilisée.

On entend par huile végétale toute huile végétale connue de l'homme du métier susceptible d'être utilisée dans une composition cosmétique.

De préférence, l'huile végétale selon l'invention est extraite de graines, ou de fruits, notamment par pressage ou par extraction au CO₂ supercritique.

De préférence, l'huile végétale selon l'invention est naturelle. On entend par huile naturelle, une huile provenant d'un végétal, en particulier d'un fruit ou de ses graines.

De préférence, l'huile végétale selon l'invention est biologique. On entend par huile biologique, une huile étant issue au moins à 95% de l'agriculture biologique.

A titre d'exemple d'huile végétale selon l'invention, il est possible de citer l'huile de prune, l'huile de pistache, l'huile d'amande, l'huile d'abricot, l'huile d'argan, l'huile d'avocat, l'huile de noix de coco, l'huile d'olive, l'huile de macadamia, l'huile de jojoba, l'huile de noisette, l'huile d'argousier, l'huile de figue, l'huile de framboise, l'huile de sésame, l'huile d'avoine, l'huile de mirabelle, l'huile de chanvre, et l'huile de date du désert.

De préférence, l'huile végétale selon l'invention est sélectionnée dans le groupe constitué de l'huile de prune, l'huile de pistache, l'huile d'amande, l'huile d'abricot, l'huile d'argan, l'huile d'avocat, l'huile de noix de coco, l'huile d'avoine, et l'huile d'olive.

De préférence, le beurre selon l'invention comprend entre 20 et 98%, plus préférablement entre 30 et 90% en masse d'huile végétale hydrogénée par rapport à la masse totale du beurre.

De préférence, le beurre selon l'invention comprend entre 2 et 80%, plus préférablement entre 10 et 70% en masse d'huile végétale par rapport à la masse totale de beurre.

De préférence, le beurre de prune, le beurre de pistache, le beurre d'amande, le beurre d'abricot, le beurre d'argan, le beurre d'avocat, le beurre d'avoine, et le beurre d'olive selon l'invention comprend entre 45 et 80%, de préférence entre 50 et 72% en masse d'huile végétale et entre 20 et 55%, de préférence entre 30 et 50% en masse de l'huile végétale hydrogénée par rapport à la masse totale de beurre.

De préférence, le beurre de noix de coco selon l'invention comprend entre 2 et 20%, de préférence entre 5 et 15 % en masse d'huile végétale et entre 80 et 98%, de préférence entre 85 et 95% en masse de l'huile végétale hydrogénée par rapport à la masse totale de beurre.

De préférence, le beurre selon l'invention comprend en outre de la vitamine E. De préférence, le beurre selon l'invention comprend entre 0,1 et 0,5%, plus préférablement entre 0,2 et 0,3% en masse de vitamine E par rapport à la masse totale de beurre.

De préférence, le beurre de prune selon l'invention comprend entre 32 et 54%, plus préférablement entre 37 et 48% en masse d'huile de prune hydrogénée et entre 46 et 68%, plus préférablement entre 52 et 63% en masse d'huile de prune par rapport à la masse totale de beurre de prune. De préférence, le beurre de prune selon l'invention comprend environ 0,2% en masse de vitamine E par rapport à la masse totale de beurre de prune.

De préférence, le beurre de pistache selon l'invention comprend entre 30 et 50%, plus préférablement entre 34 et 46 % en masse d'huile de pistache hydrogénée et entre 50 et 70%, plus préférablement entre 54 et 66% en masse d'huile de pistache par rapport à la masse totale de beurre de pistache. De préférence, le beurre de pistache selon l'invention comprend environ 0,3% en masse de vitamine E par rapport à la masse totale de beurre de pistache.

De préférence, le beurre d'amande selon l'invention comprend entre 30 et 50%, plus préférablement entre 34 et 46% en masse d'huile d'amande hydrogénée et entre 50 et 70%, plus préférablement entre 54 et 66% en masse d'huile d'amande par rapport à la masse totale de beurre d'amande. De préférence, le beurre d'amande selon l'invention comprend environ 0,2% en masse de vitamine E par rapport à la masse totale de beurre d'amande.

De préférence, le beurre d'abricot selon l'invention comprend entre 25 et 45%, plus préférablement entre 29 et 41% en masse d'huile d'abricot hydrogénée et entre 55 et 75%, plus préférablement entre 59 et 71% d'huile d'abricot par rapport à la masse totale de beurre d'abricot. De préférence le beurre d'abricot selon l'invention comprend environ 0,2% en masse de vitamine E par rapport à la masse totale de beurre d'abricot.

De préférence, le beurre d'argan selon l'invention comprend entre 35 et 55%, plus préférablement entre 39 et 51% en masse d'huile d'argan hydrogénée et entre 45 et 65%, plus préférablement entre 49 et 61% d'huile d'argan parrapport à la masse totale de beurre d'argan. De préférence, le beurre d'argan selon l'invention comprend environ 0,3% en masse de vitamine E par rapport à la masse totale de beurre d'argan.

De préférence, le beurre d'avocat selon l'invention comprend entre 20 et 40%, plus préférablement entre 27 et 38% en masse d'huile d'avocat hydrogénée et entre 60 et 80%, plus préférablement entre 62 et 73% en masse d'huile d'avocat par rapport à la masse totale de beurre d'avocat. De préférence, le beurre d'avocat selon l'invention comprend environ 0,2% en masse de vitamine E par rapport à la masse totale de beurre d'avocat.

De préférence, le beurre d'avoine selon l'invention comprend entre 35 et 55%, plus préférablement entre 40 et 50% en masse d'huile d'avoine hydrogénée et entre 45 et 65%, plus préférablement entre 50 et 60% en masse d'huile d'avoine par rapport à la masse totale de beurre d'avoine. De préférence, le beurre d'avoine comprend environ 0.2% en masse de vitamine E par rapport à la masse totale de beurre d'avoine.

De préférence, le beurre de noix de coco selon l'invention comprend entre 80 et 98%, plus préférablement entre 85 et 95% en masse d'huile de noix de coco hydrogénée et entre 2 et 20%, plus préférablement entre 5 et 15% en masse d'huile de noix de coco par rapport à la masse totale de beurre de noix de coco. De préférence, le beurre de noix de coco selon l'invention comprend environ 0,2% en masse de vitamine E par rapport à la masse totale de beurre de noix de coco.

De préférence, le beurre d'olive selon l'invention comprend entre 30 et 50%, plus préférablement entre 37 et 48% en masse d'huile d'olive hydrogénée et entre 50 et 70%, plus préférablement entre 52 et 63% en masse d'huile d'olive par rapport à la masse totale de beurre d'olive. De préférence, le beurre d'olive selon l'invention comprend environ 0,2% en masse de vitamine E par rapport à la masse totale de beurre d'olive.

De préférence, le beurre selon l'invention a un point de fusion compris entre 25 et 70°C. Le point de fusion du beurre selon l'invention peut être mesuré par n'importe quelle méthode bien connue de l'homme du métier. A titre d'exemple, le point de fusion du beurre selon l'invention peut être mesuré par calorimétrie différentielle à balayage.

A titre d'exemple, le beurre d'amande, le beurre d'abricot, le beurre d'argan, le beurre d'avocat, le beurre de prune, le beurre de pistache, le beurre d'avoine et le beurre d'olive selon l'invention ont un point de fusion compris entre 50 et 70°C, de préférence entre 55 et 70°C.

A titre d'exemple, le beurre de noix de coco selon l'invention a un point de fusion compris entre 26 et 35°C, de préférence entre 30 et 35°C.

De préférence, le beurre selon l'invention présente une viscosité d'au moins 3000 mPa.s mesurée au point de cristallisation du beurre.

De préférence également, le beurre selon l'invention présente une viscosité comprise entre 3000 et 81500 mPa.s, plus préférablement entre 3000 et 25000 mPa.s, encore plus préférablement entre 3000 et 10000 mPa.s, mesurée au point de cristallisation du beurre.

La viscosité peut être mesurée selon n'importe quelle méthode bien connue de la personne du métier. De préférence, la viscosité des beurres selon l'invention est mesurée à l'aide d'un rhéomètre. A titre d'exemple de rhéomètre il est possible de citer le rhéomètre Physica MCR 301 de la marque Anton Paar. De préférence la mesure de viscosité est effectué à une température de 80°C à 20° avec un taux de cisaillement de 38,2 s⁻¹.

Le tableau 1 ci-dessous est donné à titre d'exemple de viscosités de beurres selon l'invention :

**Tableau 1**

| Beurre selon l'invention | Point de cristallisation (°C) | Viscosité (mPa.s) mesurée au point de cristallisation |
|---|---|---|
| Beurre de prune | 32,3 | 24 000 |
| Beurre de noisette | 35,5 | 81 100 |
| Beurre d'avoine | 41,8 | 9 140 |
| Beurre de pistache | 41,8 | 6 120 |
| Beurre d'amande | 38,6 | 6 160 |
| Beurre d'abricot | 26 | 20 500 |
| Beurre d'argan | 32,3 | 12 300 |
| Beurre d'avocat | 41,8 | 8 030 |
| Beurre de chanvre | 38,6 | 3 280 |
| Beurre d'olive | 35,5 | 6 630 |

De préférence, le beurre selon l'invention a un indice de peroxyde, exprimé en mEq O2/kg, inférieur ou égale à 10, de préférence compris entre 0,1 et 5. L'indice de peroxyde correspond au degré d'oxydation des acides gras insaturés de l'échantillon. L'indice de peroxyde peut être mesuré selon n'importe quelle méthode bien connue de l'homme du métier. A titre d'exemple de méthode pour mesurer l'indice de peroxyde il est possible d'utiliser la méthode normalisée NF EN ISO 3960. Cette méthode repose sur le titrage de l'iode libéré par l'iodure de potassium en milieu acide.

De préférence, le beurre selon l'invention a un indice d'iode, exprimé en g I_{2/}100g, compris entre 0 et 82. L'indice d'iode correspond à la masse de diiode capable de se fixer sur les insaturations des acides gras contenus dans cent grammes de matière grasse. L'indice d'iode peut être mesuré selon n'importe quelle méthode bien connue de l'homme du métier. A titre d'exemple de méthode pour mesurer l'indice d'iode il est possible d'utiliser la méthode normalisée NF ISO 3961. Cette méthode repose sur le titrage de l'iode libéré par l'iodure de potassium.

A titre d'exemple, le beurre d'amande, le beurre d'abricot, le beurre d'argan, le beurre d'avocat, le beurre de prune, le beurre de pistache, le beurre d'avoine, et le beurre d'olive selon l'invention ont un indice d'iode compris entre 40 et 80, de préférence entre 45 et 75.

A titre d'exemple, le beurre de noix de coco selon l'invention a un indice d'iode compris entre 0 et 3, de préférence, inférieur à 1.

De préférence, le beurre selon l'invention a un indice d'acide, exprimé en mg KOH/g, inférieur ou égale à 7, de préférence compris entre 0,1 et 5. L'indice d'acide peut être mesuré par n'importe quelle méthode bien connue de l'homme du métier. A titre d'exemple de méthode pour mesurer l'indice d'acide, il est possible de citer la méthode normalisée NF EN ISO 660. Le principe de cette méthode repose sur le dosage de l'acidité de l'échantillon dissout dans une solution alcoolique par une solution éthanolique d'hydroxyde de potassium.

De préférence, le beurre selon l'invention a un taux de cendres, exprimé en % de cendre, inférieur ou égale à 0,1, de préférence compris entre 0,005 et 0,01. A titre d'exemple de méthode de détermination du taux de cendres, il est possible de citer la méthode normalisée NF ISO 6884. Le principe de cette méthode repose sur l'incinération de l'échantillon à une température comprise entre 550°C et 660°C jusqu'à ce qu'il soit exempt de particules charbonneuses. On procède ensuite à la pesé du résidu minéral obtenu correspondant aux cendres.

De préférence, le beurre selon l'invention est destiné à être utilisé en tant que cosmétique c'est-à-dire directement sur la peau ou les cheveux, ou dans une composition cosmétique, notamment pour nettoyer, hydrater, et protéger la peau et les cheveux ou encore pour donner du corps à la composition cosmétique.

Avantageusement, le beurre selon l'invention présente au moins l'une des propriétés suivantes : nourrissante, hydratante, cicatrisante, calmante, apaisante, adoucissante, de régénération de la peau, d'amélioration de l'élasticité de la peau.

### Procédé de préparation de beurre

De préférence, le procédé de préparation de beurre selon l'invention comprend au moins une étape d'hydrogénation d'une huile végétale selon l'invention.

L'hydrogénation d'huile végétale est bien connue de l'homme du métier. On entend par hydrogénation le procédé par lequel de l'hydrogène est ajouté sur les doubles liaisons de composés organiques insaturés, en particulier les acides gras qui composent les huiles végétales. Typiquement, l'huile est mise à réagir avec de l'hydrogène à température et pression élevées en présence d'un catalyseur. L'huile est maintenue sous agitation dans un réacteur fermé en présence d'un catalyseur, dans lequel est injecté de l'hydrogène en discontinu sous une pression comprise entre 1 et 5 bars à une température comprise entre 100 et 250°C, plus préférablement entre 110 et 180°C. Avantageusement et de manière inattendue, l'hydrogénation d'une huile végétale selon l'invention à une température telle que définie ci-dessus permet d'obtenir un beurre ayant les propriétés requises pour une utilisation dans des compositions cosmétiques.

De préférence, la source de dihydrogène pour la réaction d'hydrogénation selon l'invention est du dihydrogène gazeux.

De préférence, la réaction d'hydrogénation selon l'invention est réalisée à une température comprise entre 100 et 250°C, de préférence entre 180°C et 210°C, et à une pression comprise entre 1 et 5 bars.

De préférence, la réaction d'hydrogénation selon l'invention est effectuée en présence d'un catalyseur. De préférence, le catalyseur est un catalyseur hétérogène A titre d'exemple de catalyseur convenant pour la réaction d'hydrogénation selon l'invention, il est possible de citer les métaux du groupe du platine, en particulier le platine, le palladium, le rhodium et le ruthénium, les catalyseurs à base de nickel, tel que le nickel de Raney ou le nickel d'Urushibara et les catalyseurs à base de cobalt, de fer et de chromite de cuivre ou de zinc. De préférence, le catalyseur utilisé est un catalyseur de type nickel de Raney.

De préférence, la quantité de catalyseur utilisée dans la réaction d'hydrogénation selon l'invention est comprise entre 0,01 et 2%, de préférence entre 0,01 et 0,5% en masse par rapport à la masse de l'huile végétale que l'on souhaite hydrogénée.

De préférence, la réaction d'hydrogénation de l'huile végétale selon l'invention a pour effet de rendre l'huile végétale solide ou semi-solide.

De préférence, le procédé selon l'invention comprend une étape d'ajout d'huile végétale à l'huile végétale hydrogénée. De préférence, l'huile végétale ajoutée est identique à l'huile végétale ayant été hydrogénée. De préférence, l'huile végétale est ajoutée lentement à l'huile hydrogéné encore chaude dans le réacteur. De préférence, l'ajout d'huile végétale se fait sous flux d'azote et sous agitation. Le mélange est homogénéisé sous agitation pendant une durée d'au moins 30 minutes et la température est abaissée. De préférence, cette étape permet d'obtenir la consistance d'un beurre.

La quantité d'huile végétale hydrogénée selon l'invention est de préférence comprise entre 20 et 98%, plus préférablement entre 30 et 90% en masse par rapport à la masse totale de beurre.

De préférence, la quantité d'huile végétale ajoutée est comprise entre 2 et 80% en masse, de préférence entre 10 et 70% par rapport à la masse totale de beurre.

De préférence, le beurre préparé ou susceptible d'être préparé par le procédé selon l'invention comprend entre 20 et 98%, plus préférablement entre 30 et 90% en masse d'huile végétale hydrogénée par rapport à la masse totale du beurre.

De préférence, le beurre préparé ou susceptible d'être préparé par le procédé comprend entre 2 et 80%, plus préférablement entre 10 et 70% en masse d'huile végétale par rapport à la masse totale de beurre.

A titre d'exemple, l'huile de prune, l'huile de pistache, l'huile d'amande, l'huile d'abricot, l'huile d'argan, l'huile d'avocat, l'huile d'avoine, et l'huile d'olive est ajouté à l'huile hydrogénée correspondante en une quantité comprise entre 45 et 80%, de préférence entre 50 et 72% en masse par rapport à la masse totale de beurre.

A titre d'exemple, l'huile de noix de coco est ajoutée à l'huile de noix de coco hydrogénée en une quantité comprise entre 2 et 20%, de préférence entre 5 et 15% en masse par rapport à la masse totale de beurre.

De préférence, le procédé selon l'invention comprend au moins une étape de désodorisation.

Selon un mode de réalisation de l'invention, l'étape de désodorisation est effectuée sur l'huile végétale hydrogénée selon l'invention. Le beurre obtenu selon l'invention a ainsi l'odeur de l'huile végétale ajouté l'huile végétale hydrogénée désodorisée.

Selon un autre mode de réalisation de l'invention, l'étape de désodorisation est réalisée sur le beurre selon l'invention c'est-à-dire après l'étape d'ajout d'huile végétale à l'huile végétale hydrogénée.

La désodorisation consiste en l'élimination des substances odorantes. La désodorisation peut être réalisée selon n'importe quelle méthode bien connue de l'homme du métier. Typiquement, la désodorisation repose sur l'injection d'un flux de vapeurd'eau ou d'azote dans l'huile chauffée à une température comprise entre 150 et 250°C sous vide compris entre 1 et 20 mbar, de préférence entre 2 et 6 mbar. Les substances volatiles responsables des odeurs de l'huile sont séparés grâce au flux puis éliminées à l'aide d'une pompe à vide et un refroidissement.

De préférence, la désodorisation selon l'invention est réalisée à une température comprise entre 160 et 230°C, de préférence sous vide compris entre 2 et 20 mbar, de préférence d'environ 5mbar. La désodorisation selon l'invention peut être réalisée sous un flux de vapeur d'eau ou sous un flux d'azote, pendant une durée de préférence de moins de trois heures, plus préférablement de moins de deux heures et demie.

De préférence, le procédé de préparation de beurre sélectionné parmi le beurre de noisette, le beurre de mirabelle, et le beurre de prune comprend une étape de désodorisation de l'huile végétale hydrogénée. Avantageusement le beurre obtenu a l'odeur de l'huile végétale ajoutée à l'huile végétale hydrogénée désodorisée.

De préférence, le procédé selon l'invention comprend en outre une étape d'ajout de vitamine E. De préférence, la quantité de vitamine E ajoutée au beurre est comprise entre 0,1 et 0,5 % en masse par rapport à la masse totale de beurre. Plus préférablement, la quantité de vitamine E ajoutée au beurre est comprise entre 0,2 et 0,3 % en masse par rapport à la masse totale de beurre.

De préférence, le beurre selon l'invention destiné à être utilisé dans des compositions cosmétiques est préparé par le procédé selon l'invention tel que défini ci-dessus.

### Compositions cosmétiques

De préférence, au moins un beurre selon l'invention est utilisé dans des compositions cosmétiques comme ingrédient cosmétique, notamment en tant qu'agent de texture, agent nourrissant, agent émolliant, conditionneur, ou pour apporter un effet fondant.

De préférence, la composition cosmétique selon l'invention comprend au moins un beurre selon l'invention, sélectionné dans le groupe constitué du beurre d'amande, du beurre d'abricot, du beurre d'argan, du beurre d'avocat, du beurre de noix de coco, du beurre d'olive, du beurre de macadamia, du beurre de jojoba, du beurre de noisette, du beurre d'argousier, du beurre de figue, du beurre de framboise, du beurre de sésame, du beurre d'avoine, du beurre de mirabelle, du beurre de date de désert, du beurre de prune, du beurre de pistache, du beurre de chanvre, et de leur mélange.

De préférence, la composition cosmétique selon l'invention comprend au moins un beurre selon l'invention sélectionné dans le groupe constitué du beurre d'amande, du beurre d'abricot, du beurre d'argan, du beurre d'avocat, du beurre de noix de coco, du beurre de prune, du beurre de pistache, du beurre d'avoine, du beurre d'olive et de leur mélange.

Les compositions cosmétiques selon l'invention peuvent être de n'importe quelle forme bien connue de l'homme du métier. A titre d'exemple, les compositions cosmétiques selon l'invention peuvent être sous la forme de crème, de lait, de beurre, d'émulsion, de baume, de stick, de gel, de solide, d'huile, ou toute autre forme bien connue de l'homme du métier.

De préférence, les compostions cosmétiques comprenant au moins un beurre selon l'invention sont sélectionnées dans le groupe constitué des produits d'hygiène tels que les gels douche, gels nettoyant intime, nettoyants visage, démaquillants, savons, shampoing, shampoing solide, déodorants ; des produits de soin de visage tels que les crèmes anti-rides, crèmes de jour, crèmes de nuit, crèmes hydratantes, masques pour le visage ou le contour des yeux, baumes pour les lèvres, huiles pour le visage; des produits capillaires tel que les après-shampoing, masques pour les cheveux ou le cuir chevelu, huiles pour les cheveux, gels, crèmes traitantes pour les cheveux secs à pointes abimées, produits d'entretien de la barbe ; des produits de maquillage tels que du fond de teint, du rouge à lèvre, des CC crèmes, des BB crèmes, des anticernes, des produits autobronzants, des enlumineurs de teint, des matifiants ; des produits solaires tels que des crèmes, huiles ou lotions solaires ou après-solaires ; des produits de soins pour le corps tels que les huiles, baumes, crèmes ou laits pour le corps, des crèmes pour les mains, des crèmes anti-vergetures ; et des produits de massage tels que les huiles, et baumes de massage.

Les compositions cosmétiques selon l'invention peuvent en outre comprendre au moins un composé additionnel sélectionné dans le groupe constitué d'un émulsionnant, d'un agent de texture, d'un émollient, d'un solvant, d'un humectant, d'un gélifiant, d'un conservateur, d'un régulateur de pH, d'un agent texturant, d'un colorant, d'un parfum, d'un tampon, d'un antioxydant, d'un pigment, d'un agent de durcissement, d'une charge, d'un tensio-actif, d'un lubrifiant, d'un conditionneur capillaire, d'un anti-irritant, d'un actif anti-inflammatoire, d'un agent anti-tiraillement d'un matifiant, et d'un co-émulsionnant.

La présente invention est illustrée par les exemples non limitatifs suivants.

### EXEMPLES

### Exemple 1 : préparation de beurre d'avocat

### 1.1. Hydrogénation

L'huile d'avocat est introduite dans un réacteur sous pression de 300 l à une température de 100°C. Un catalyseur hétérogène d'hydrogénation de type Nickel de Raney sur charbon (PRICAT 9910^{®} JOHNSON MATTEY, 22% de Ni) est également introduit dans le réacteur.

La température du réacteur est élevée et maintenue à 210°C afin de conserver une température de coeur de réaction de 180°C. 2 bars d'hydrogène sont introduits dans le réacteur. Le mélange est agité pendant 15 minutes. L'agitation est stoppée et l'hydrogène est dégazé lentement jusqu'à atteindre une pression nulle. 5 bars d'hydrogène sont réintroduits dans le réacteur. L'agitation est lancée et la consommation d'hydrogène est suivie. Lorsqu'il n'y a plus de consommation sur le manomètre de la bouteille d'hydrogène la température du réacteur est abaissée à 110°C. L'hydrogène est dégazé lentement.

### 1.2. Formulation

Dans le réacteur de 300 l, l'huile d'avocat est ajoutée lentement à l'huile d'avocat hydrogénée encore chaude (110°C) sous un flux d'azote afin de limiter l'oxydation de l'huile. L'agitation est lancée et le mélange est homogénéisé pendant 30 minutes. La température est abaissée à 90°C. De la vitamine E (BIOXAN T90) est ajoutée. Un filtre presse de 30 l à cartouches est connecté à la sortie du réacteur et l'huile est filtrée.

Le tableau 2 ci-dessous présente les proportions d'huile d'avocat hydrogénée et d'huile d'avocat dans le beurre d'avocat.

**[Tableau 2]**

| % en masse d'huile hydrogénée par rapport à la masse totale de beurre | % en masse d'huile par rapport à la masse totale de beurre | % en masse de vitamine E par rapport à la masse totale de beurre |
|---|---|---|
| 30-35 | 65-70 | 0,2-0,3 |

### 1.3. Désodorisation

Le mélange est désodorisé en élevant la température à 160°C sous un vide de 5 mbar sous un flux de vapeur d'eau pendant 1h30. Le beurre désodorisé ainsi obtenu est refroidi à 70°C.

### 1.4. Caractérisation

Le point de fusion du beurre est mesuré. L'indice de peroxyde, exprimé en mEq O2/kg, est mesuré selon la méthode normalisée NF EN ISO 3960. L'indice d'iode, exprimé en g I_{2/}100g est mesuré selon la méthode normalisée NF ISO 3961. L'indice d'acide, exprimé en mg KOH/g, est mesuré selon la méthode normalisée NF EN ISO 660. Le taux de cendres, exprimé en % de cendre, est mesuré selon la méthode normalisée NF ISO 6884.

Les résultats sont présentés dans le tableau 3 suivant :

**[Tableau 3]**

| Point de fusion | Indice de peroxyde (mEq O₂/kg) | Indice d'acide (mg KOH/g) | Indice d'iode (g 12/100g) | Cendres (% de cendres) |
|---|---|---|---|---|
| 58-64 | < 10 | ≤ 5 | 47-63 | ≤ 0,1 |

Le profil d'acides gras du beurre d'avocat est présenté dans le tableau 4 ci-dessous :

**[Tableau 4]**

| **Acide gras** | **% d'acide gras dans le beurre** |
|---|---|
| Acide linoléique | 4,0-12,0 |
| Acide linolénique | 0,0-2,0 |
| Acide oléique | 30,0-50,0 |
| Acide palmitique | 12,0-28,0 |
| Acide palitoléique | 0,0-10,1 |
| Acide stéarique | 15,0-35,0 |

### Exemple 2 : crème traitante pour les cheveux secs à pointes abimées

La composition d'une crème pour les cheveux secs à pointes abimées comprenant le beurre d'avocat selon l'invention, est présentée dans le tableau 5 ci-dessous.

**[Tableau 5]**

| **Nom** | **% en masse par rapport à la masse totale de produit** |
|---|---|
| Alcool cétéarylique 60%, dipalmitoylethy hydroxyéthylamonium méthosulfate 25%, ceteareth-20 15% | 6 |
| Alcool cétéarylique | 2 |
| Beurre d'avocat selon l'invention | 2 |
| Huile de noyer désodorisée | 5 |
| Eau purifiée | 78,25 |
| Glycérine | 5 |
| Hydroxyéthylcellulose | 0,5 |
| Benzoate de sodium | 0,5 |
| Parfum | 0,75 |
| Acide citrique | QSP |

Le beurre d'avocat obtenu selon l'invention donne une texture riche à la crème et lui confère des propriétés nourrissantes.

### Exemple 3 : masque capillaire

Une formulation de masque capillaire comprenant du beurre d'amande et du beurre d'argan selon l'invention est présentée dans le tableau 6 ci-dessous :

**[Tableau 6]**

| **Nom** | **% en masse par rapport à la masse totale de produit** |
|---|---|
| Alcool cétostéarylique dipalmitoylethyl hydroxyethylmonium methosulfate ceteareth-20 | 8 |
| Beurre de d'amande selon l'invention | 3 |
| Beurre d'argan selon l'invention | 2 |
| Eau purifiée | 80.49 |
| Glycérine | 5 |
| Benzoate de sodium | 0.50 |
| Colorant | 0.0013 |
| Colorant | 0.0024 |
| Parfum | 1.00 |
| Acide citrique | QSP |

Le beurre d'amande selon l'invention protège les cheveux et leur donne de la douceur. Le beurre d'argan selon l'invention nourrit et hydrate les cheveux et leur apporte de la souplesse.

## Revendications

1. Beurre comprenant une huile végétale et ladite huile végétale hydrogénée.

2. Beurre selon la revendication 1, comprenant entre 2 et 80% en masse d'huile végétale par rapport à la masse totale de beurre et entre 20 et 98% en masse de ladite huile végétale hydrogénée par rapport à la masse totale du beurre.

3. Beurre selon la revendication 1 ou 2, comprenant en outre entre 0,2 et 0,3% en masse de vitamine E par rapport à la masse totale de beurre.

4. Beurre selon l'une des revendications 1 à 3, sélectionné dans le groupe constitué du beurre de prune, du beurre de pistache, du beurre d'amande, du beurre d'abricot, du beurre d'argan, du beurre d'avocat, du beurre d'olive, du beurre de noix de coco, et du beurre d'avoine.

5. Beurre selon la revendication 4, sélectionné dans le groupe constitué du beurre de prune, du beurre de pistache, du beurre d'amande, du beurre d'abricot, du beurre d'argan, du beurre d'avocat, du beurre d'avoine, et du beurre d'olive et comprenant entre 20 et 55% en masse d'huile végétale hydrogénée et entre 45 et 80% en masse d'huile végétale par rapport à la masse totale de beurre.

6. Beurre selon la revendication 4, étant un beurre de noix de coco et comprenant entre 2 et 20 % en masse d'huile végétale de noix de coco et entre 80 et 98% en masse de l'huile végétale hydrogénée de noix de coco par rapport à la masse totale de beurre.

7. Procédé de préparation de beurre destiné à être utilisé dans des compositions cosmétiques à partir d'une huile végétale comprenant les étapes suivantes :
- une étape d'hydrogénation de l'huile végétale ;
- une étape d'ajout de l'huile végétale à l'huile végétale hydrogénée de sorte à obtenir un beurre ;
- éventuellement une étape de désodorisation.

8. Procédé selon la revendication 7, dans lequel l'étape de désodorisation est effectuée sur l'huile végétale hydrogénée.

9. Procédé selon la revendication 7, dans lequel l'étape de désodorisation est effectuée sur le beurre.

10. Procédé selon l'une quelconque des revendications 7 à 9, comprenant en outre au moins une étape d'ajout de vitamine E.

11. Procédé selon l'une quelconque des revendications 7 à 10, dans lequel l'huile végétale est sélectionnée dans le groupe constitué de l'huile de prune, l'huile de pistache, l'huile d'amande, l'huile d'abricot, l'huile d'argan, l'huile d'avocat, l'huile de noix de coco, l'huile d'avoine et l'huile d'olive.

12. Beurre destiné à être utilisé dans des compositions cosmétiques susceptible d'être préparé selon le procédé défini dans les revendications 7 à 11.

13. Composition cosmétique comprenant au moins un beurre tel que défini dans l'une des revendications 1 à 6 ou 12.

14. Composition cosmétique selon la revendication 13, sous la forme de crème, de lait, de beurre, d'émulsion, de baume, de stick, de gel, de solide, ou d'huile.

15. Composition cosmétique selon la revendication 13 ou 14, sélectionnée dans le groupe constitué des produits d'hygiène, des produits de soin de visage, des produits capillaires, des produits de maquillage, des produits solaires, des produits de soins pour le corps, et des produits de massage.

16. Composition cosmétique selon l'une des revendications 13 à 15, comprenant en outre au moins un composé additionnel sélectionné dans le groupe constitué d'un émulsionnant, d'un agent de texture, d'un émollient, d'un solvant, d'un humectant, d'un gélifiant, d'un conservateur, d'un régulateur de pH, d'un agent texturant, d'un colorant, d'un parfum, d'un tampon, d'un antioxydant, d'un pigment, d'un agent de durcissement, d'une charge, d'un tensio-actif, d'un lubrifiant, d'un conditionneur capillaire, d'un anti-irritant, d'un matifiant, et d'un co-émulsionnant.
